# EUROPEAN PATENT SPECIFICATION

(11) **EP 1 595 894 B1**
(45) Date of publication and mention of the grant of the patent: **12.10.2011**
(21) Application number: 04711754.4
(22) Date of filing: 17.02.2004
(51) Int. Cl.: C08F 4/00, C08F 20/00

(54) **PROCESS FOR THE PRODUCTION OF LIVING RADICAL POLYMERS**
VERFAHREN ZUR HERSTELLUNG VON LEBENDEN RADIKALISCHEN POLYMEREN
PROCEDE POUR LA PRODUCTION DE POLYMERES A RADICAUX VIVANTS

(30) Priority: 17.02.2003 JP 2003038590; 24.09.2003 JP 2003331544
(43) Date of publication of application: 16.11.2005
(73) Proprietor: OTSUKA CHEMICAL COMPANY, LIMITED, Osaka-shi, Osaka 540-0021 (JP)
(72) Inventor: YAMAGO, Shigeru, Kyoto-shi, Kyoto 606-8152 (JP); YOSHIDA, Junichi, Hirakata-shi, Osaka 573-0076 (JP); KAMESHIMA, Takashi, Tokushima-shi, Tokushima 771-0193 (JP)
(74) Representative: Ritthaler, Wolfgang
(86) International application number: PCT/JP2004/001707
(87) International publication number: WO 2004/072126

(56) References cited:
- WO-A1-2004/014848
- WO-A1-2004/014962
- TAKAGI K ET AL: "CONTROLLED RADICAL POLYMERIZATION OF STYRENE UTILIZING EXCELLENT RADICAL CAPTURING ABILITY OF DIPHENYL DITELLURIDE" POLYMER BULLETIN, SPRINGER, HEIDELBERG, DE, vol. 43, no. 2/3, September 1999 (1999-09), pages 143-150, XP000865402 ISSN: 0170-0839
- DATABASE CA [Online] CHEMICAL ABSTRACTS SERVICE, COLUMBUS, OHIO, US; YAMAKO, SHIGERU ET AL YAMAKO, SHIGERU ET AL: "Manufacture of organotellurium compounds as living radical polymerization initiators Manufacture of organotellurium compounds as living radical polymerization initiators" XP002394192 retrieved from STN Database accession no. 2004:986149 & JP 2004 323437 A (OTSUKA CHEMICAL CO., LTD., JAPAN OTSUKA CHEMICAL CO., LTD., JAPAN) 18 November 2004 (2004-11-18)
- GOTO A. ET AL: 'Mechanism-based invention of high-speed living radical polymerization using organotellurium compounds and azo-initiators' JACS vol. 125, no. 29, 23 July 2003, pages 8720 - 8721, XP002979332
- YAMAGO S. ET AL: 'Tailored synthesis of structurally defined polymers by organotellurium-mediated living radical polymerization (TERP)' JACS vol. 124, no. 46, 20 November 2002, pages 13666 - 13667, XP002964725
- YAMAGO S. ET AL: 'Organotellurium compounds as novel initiators for controlled/living radical polymerizations' JACS vol. 124, no. 12, 27 March 2002, pages 2874 - 2875, XP002964726

## Description

### SPECIFICATION

### TECHNICAL FIELD

The present invention relates to a process for producing living radical polymers.

### BACKGROUND ART

Living radical polymerization is a polymerization process which is adapted for precision control of molecular structures while ensuring convenience and universal usefulness of radical polymerization, and is powerful means for preparing novel polymer materials. Georges et al. has made a report on a typical example of living radical polymerization using TEMPO (2,2,6,6-tetramethyl-1-piperidinyloxy) as an initiator (see, e.g., JP-A-1994-199916 (Claim 1, Examples I ~X).

Takagi et al. (Polymer Bulletin 43, 143-150; 1999) discloses the controlled radical polymerization of styrene in the presence of diphenyl ditelluride and azobisisobutyronitrile.

### DISCLOSURE OF THE INVENTION

The process of JP-A-1994-199916 makes it possible to control molecular weights and molecular weight distributions, but requires a high polymerization temperature and is difficult to use for monomers having a thermally unstable functional group. The process is also unsuited to the control of modification of terminal functional groups of high molecular weight compounds. Namely, all of the examples of JP-A-1994-199916 are concerned with a polymerization of styrene except two examples of copolymerization of styrene and myrcene.

Styrene is polymerized at a high temperature of 123 to 150°C for more than 7 hours, and in some cases, for 12.9 to 69 hours with conversion of 86 to 92 % and PD of 1.2 to 1.4. Thus, the polymerization of patent literature 1 requires at least 7 hours ever at a high temperature of more than 123°C with less than 92 % in conversion.

An object of the present invention is to provide a process for producing a living radical polymer by polymerizing a vinyl monomer with use of an organotellurium compound represented by the formula (1) and an azo type polymerization initiator, the process making possible precision control of molecular weights and molecular weight distributions (PD=Mw/Mn), and the polymer.

An object of the present invention is to provide a process for producing a living radical polymer under mild conditions, in a short period of time and a high yield, the process making possible precision control of molecular weights and molecular weight distributions (PD=Mw/Mn), and the polymer.

The present invention provides a process for producing a living radical polymer characterized in that a vinyl monomer is polymerized with use of an organotellurium compound represented by the formula (1) and an azo type polymerization initiator, 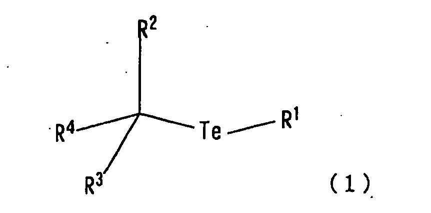 wherein R¹ is C₁-C₈ alkyl, substituted aryl or an aromatic heterocyclic group, R² and R³ are each a hydrogen atom or C₁-C₈ alkyl, and R⁴ is aryl, substituted aryl, an aromatic heterocyclic group, acyl, oxycarbonyl or cyano.

The invention provides a process for preparing living radical polymers which realizes precision control of molecular weights and molecular weight distributions under mild conditions and in a short period of time. The living radical polymers obtained by the polymerization process of the invention readily permit conversion of terminal groups to other functional groups, are useful for preparing macromonomers and useful as crosslinking sites and are usable as compatibilizing agents and as materials for block polymers.

The living radical polymer of the present invention is produced by polymerizing a vinyl monomer with use of an organotellurium compound represented by the formula (1) and an azo type polymerization initiator 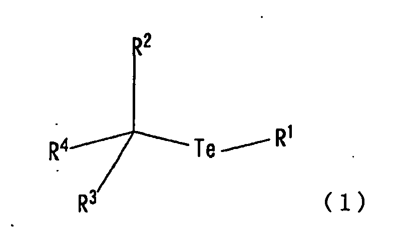 wherein R¹ is C₁-C₈ alkyl, substituted aryl or an aromatic heterocyclic group, R² and R³ are each a hydrogen atom or C₁-C₈ alkyl, and R⁴ is aryl, substituted aryl, an aromatic heterocyclic group, acyl, oxycarbonyl or cyano.

The organotellurium compound to be used in the present invention is a compound represented by the formula (1).

Examples of groups represented by R¹ are as follows.

Examples of C₁-C₈ alkyl groups usable are straight-chain, branched chain or cyclic alkyl groups having 1 to 8 carbon atoms, such as methyl, ethyl, n-propyl, isopropyl, cyclopropyl, n-butyl, sec-butyl, tert-butyl, cyclobutyl, n-pentyl, n-hexyl, n-heptyl and n-octyl. Preferable alkyl groups are straight-chain or branched chain alkyl groups having 1 to 4 carbon atoms. Methyl, ethyl or n-butyl is more preferable.

Examples of groups usable include substituted aryl groups such as phenyl having a substituent and naphthyl having a substituent, and aromatic heterocyclic groups such as pyridyl, pyrrol, furyl and thienyl. Examples of substituents of aryl groups having a substituent are a halogen atom, hydroxyl, alkoxyl, amino, nitro, cyano, carbonyl-containing groups represented by -COR⁵ (R⁵ = C₁-C₈ alkyl, aryl, C₁-C₈ alkoxyl or aryloxy), sulfonyl, and trifluoromethyl. A preferable substituted aryl group is trifluoromethyl-substituted phenyl. Preferably such substituted groups have one or two substituents at the para-position or ortho-position.

Examples of groups represented by R² and R³ are as follows.

Examples of C₁-C₈ alkyl groups usable are the same as the alkyl groups represented by R¹ and given above.

Examples of groups represented by R⁴ are as follows.

Examples of aryl, substituted aryl, aromatic heterocyclic groups usable are the same as those groups represented by R¹ and given above.

Examples of acyl groups usable are formyl, acetyl, and benzoyl.

Examples of preferred oxycarbonyl groups are those represented by -COOR⁶ (R⁶ = H, C₁-C₈ alkyl or aryl) such as carboxyl, methoxycarbonyl, ethoxycarbonyl, propoxycarbonyl, n-butoxycarbonyl, sec-butoxycarbonyl, tert-butoxycarbonyl, n-pentoxycarbonyl and phenoxycabonyl. Methoxycarbonyl and ethoxycarbonyl are more desirable oxycarbonyl groups.

Examples of preferred groups represented by R⁴ are aryl, substituted aryl, oxycarbonyl and cyano. The aryl group is preferably phenyl. Examples of preferred substituted aryl groups are phenyl substituted with a halogen atom and phenyl substituted with trifluoromethyl. When the substituent is a halogen, the phenyl is substituted with preferably one to five halogen atoms. In the case of alkoxyl or trifluoromethyl, preferably one or two substituents are present. When having one substituent, the group is substituted preferably at the para- or ortho-position. When the group has two substituents, the meta-positions are preferred. Examples of preferred oxycarbonyl groups are methoxycarbonyl and ethoxycarbonyl.

Examples of preferred organotellurium compounds represented by the formula (1) are compounds wherein R¹ is C₁-C₄ alkyl, R² and R³ are each a hydrogen atom or C₁-C₄ alkyl, and R⁴ is aryl, substituted aryl or oxycarbonyl. More preferable organotellurium compounds are those wherein R¹ is C₁-C₄ alkyl, R² and R³ are each a hydrogen atom or C₁-C₄ alkyl, and R⁴ is phenyl, substituted phenyl, methoxycarbonyl or ethoxycarbonyl.

Examples of organotellurium compounds represented by the formula (1) are as follows.

Such organotellurium compounds are preferably (methyltellanyl-methyl)benzene, (1-methyltellanyl-ethyl)benzene, (2-methyltellanyl-propyl)benzene, 1-chloro-4-(methyltellanyl-methyl)benzene, 1-hydroxy-4-(methyltellanyl-methyl)benzene, 1-methoxy-4-(methyltellanyl-methyl)benzene, 1-amino-4-(methyltellanyl-methyl)benzene, 1-nitro-4-(methyltellanyl-methyl)benzene, 1-cyano-4-(methyltellanyl-methyl)benzene, 1-methylcarbonyl-4-(methyltellanyl-methyl)benzene, 1-phenylcarbonyl-4-(methyltellanyl-methyl)benzene, 1-methoxycarbonyl-4-(methyltellanyl-methyl)benzene, 1-phenoxycarbonyl-4-(methyltellanyl-methyl)benzene, 1-sulfonyl-4-(methyltellanyl-methyl)benzene, 1-trifluoromethyl-4-(methyltellanyl-methyl)benzene, 1-chloro-4-(1-methyltellanyl-ethyl)benzene, 1-hydroxy-4-(1-methyltellanyl-ethyl)benzene, 1-methoxy-4-(1-methyltellanyl-ethyl)benzene, 1-amino-4-(1-methyltellanyl-ethyl)benzene, 1-nitro-4-(1-methyltellanyl-ethyl)benzene, 1-cyano-4-(1-methyltellanyl-ethyl)benzene, 1-methylcarbonyl-4-(1-methyltellanyl-ethyl)benzene, 1-phenylcarbonyl-4-(1-methyltellanyl-ethyl)benzene, 1-methoxycarbonyl-4-(1-methyltellanyl-ethyl)benzene, 1-phenoxycarbonyl-4-(1-methyltellanyl-ethyl)benzene, 1-sulfonyl-4-(1-methyltellanyl-ethyl)benzene, 1-trifluoromethyl-4-(1-methyltellanyl-ethyl)benzene [1-(1-methyltellanyl-ethyl)-4-trifluoromethylbenzene], 1-(1-methyltellanyl-ethyl)-3,5-bis-trifluoromethylbenzene, 1,2,3,4,5-pentafluoro-6-(1-methyltellanyl-ethyl)benzene, 1-chloro-4-(2-methyltellanyl-propyl)benzene, 1-hydroxy-4-(2-methyltellanyl-propyl)benzene, 1-methoxy-4-(2-methyltellanyl-propyl)benzene, 1-amino-4-(2-methyltellanyl-propyl)benzene, 1-nitro-4-(2-methyltellanyl-propyl)benzene, 1-cyano-4-(2-methyltellanyl-propyl)benzene, 1-methylcarbonyl-4-(2-methyltellanyl-propyl)benzene, 1-phenylcarbonyl-4-(2-methyltellanyl-propyl)benzene, 1-methoxycarbonyl-4-(2-methyltellanyl-propyl)benzene, 1-phenoxycarbonyl-4-(2-methyltellanyl-propyl)benzene, 1-sulfonyl-4-(2-methyltellanyl-propyl)benzene, 1-trifluoromethyl-4-(2-methyltellanyl-propyl)benzene, 2-(methyltellanyl-methyl)pyridine, 2-(1-methyltellanyl-ethyl)pyridine, 2-(2-methyltellanyl-propyl)pyridine, 2-methyl-2-methyltellanyl-propanal, 3-methyl-3-methyltellanyl-2-butanone, methyl 2-methyltellanyl-ethanate, methyl 2-methyltellanyl-propionate, methyl 2-methyltellanyl-2-methylpropionate, ethyl 2-methyltellanyl-ethanate, ethyl 2-methyltellanyl-propionate, ethyl 2-methyltellanyl-2-methylpropionate [ethyl-2-methyl-2-methyltellanyl-propionate], ethyl 2-(n-butyltellanyl)-2-methylpropionate [ethyl-2-methyl-2-n-butyltellanyl-propionate], 2-methyltellanylacetonitrile, 2-methyltellanyl-propionitrile, and 2-methyl-2-methyltellanyl-propionitrile. The above compounds also include all compounds having ethyltellanyl, 1-ethyllellanyl, 2-ethyltellanyl, butyltellanyl, 1-butyltellanyl or 2-butyltellanyl, as changed from the portion of methyltellanyl, 1-methyltellanyl or 2-methyltellanyl. Preferable are (methyltellanyl-methyl)benzene, (1-methyltellanyl-ethyl)benzene, (2-methyltellanyl-propyl)benzene, 1-chloro-4-(1-methyltellanyl-ethyl)benzene, 1-trifluoromethyl-4-(1-methyltellanyl-ethyl)benzene [1-(1-methyltellanyl-ethyl)-4-trifluoromethylbenzene], methyl 2-methyltellanyl-2-methylpropionate, ethyl 2-methyltellanyl-2-methylpropionate [ethyl-2-methyl-2-methyltellanyl-propionate], ethyl 2-(n-butyltellanyl)-2-methylpropionate [ethyl-2-methyl-2-n-butyltellanyl-propionate], 1-(1-methyltellanyl-ethyl)-3,5-bis-trifluoromethylbenzene, 1,2,3,4,5-pentafluoro-6-(1-methyltellanyl-ethyl)benzene, 2-methyltellanyl-propionitrile, 2-methyl-2-methyltellanylpropionitrile, (ethyltellanyl-methyl)benzene, (1-ethyltellanyl-ethyl)benzene, (2-ethyltellanyl-propyl)benzene, methyl 2-ethyltellanyl-2-methylpropionate, ethyl 2-ethyltellanyl-2-methylpropionate, 2-ethyltellanyl-propionitrile, 2-methyl-2-ethyltellanylpropionitrile, (n-butyltellanyl-methyl)benzene, (1-n-butyltellanyl-ethyl)benzene, (2-n-butyltellanyl-propyl)benzene, methyl 2-n-butyltellanyl-2-methylpropionate, ethyl 2-n-butyltellanyl-2-methylpropionate, 2-n-butyltellanyl-propionitrile, 2-methyl-2-n-butyltellanyl-propionitrile.

The organotellurium compound represented by the formula (1) can be prepared by reacting a compound of the formula (2), a compound of the formula (3) and metallic tellurium.

Examples of compounds represented by the formula (2) are as follows. 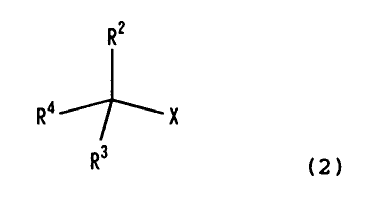 wherein R², R³ and R⁴ are as defined above, and X is a halogen atom.

Examples of groups represented by R², R³ and R⁴ are as given above.

Examples of groups represented by X can be a halogen atom such as fluorine, chlorine, bromine or iodine. Chlorine and bromine are preferable.

Examples of compounds usable are benzyl chloride, benzyl bromide, 1-chloro-1-phenylethane, 1-bromo-1-phenylethane, 2-chloro-2-phenylpropane, 2-bromo-2-phenylpropane, p-chlorobenzyl chloride, p-hydroxybenzyl chloride, p-methoxybenzyl chloride, p-aminobenzyl chloride, p-nitrobenzyl chloride, p-cyanobenzyl chloride, p-methylcarbonylbenzyl chloride, phenylcarbonylbenzyl chloride, p-methoxycarbonylbenzyl chloride, p-phenoxycarbonylbenzyl chloride, p-sulfonylbenzyl chloride, p-trifluoromethylbenzyl chloride, 1-chloro-1-(p-chlorophenyl)ethane, 1-bromo-1-(p-chlorophenyl)ethane, 1-chloro-1-(p-hydroxyphenyl)ethane, 1-bromo-1-(p-hydroxyphenyl)-ethane, 1-chloro-1-(p-methoxyphenyl)ethane, 1-bromo-1-(p-methoxyphenyl)ethane, 1-chloro-1-(p-aminophenyl)ethane, 1-bromo-1-(p-aminophenyl)ethane, 1-chloro-1-(p-nitrophenyl)ethane, 1-bromo-1-(p-nitrophenyl)ethane, 1-chloro-1-(p-cyanophenyl)ethane, 1-bromo-1-(p-cyanophenyl)ethane, 1-chloro-1-(p-methylcarbonylphenyl)ethane, 1-bromo-1-(p-methylcarbonylphenyl)ethane, 1-chloro-1-(p-phenylcarbonylphenyl)ethane, 1-bromo-1-(p-phenylcarbonylphenyl)-ethane, 1-chloro-1-(p-methoxycarbonylphenyl)ethane, 1-bromo-1-(p-methoxycarbonylphenyl)ethane, 1-chloro-1-(p-phenoxycarbonylphenyl)-ethane, 1-bromo-1-(p-phenoxycarbonylphenyl)ethane, 1-chloro-1-(p-sulfonylphenyl)ethane, 1-bromo-1-(p-sulfonylphenyl)ethane, 1-chloro-1-(p-trifluoromethylphenyl)ethane, 1-bromo-1-(p-trifluoromethylphenyl)ethane, 2-chloro-2-(p-chlorophenyl)propane, 2-bromo-2-(p-chlorophenyl)propane, 2-chloro-2-(p-hydroxyphenyl)-propane, 2-bromo-2-(p-hydroxyphenyl)propane, 2-chloro-2-(p-methoxyphenyl)propane, 2-bromo-2-(p-methoxyphenyl)propane, 2-chloro-2-(p-aminophenyl)propane, 2-bromo-2-(p-aminophenyl)propane, 2-chloro-2-(p-nitrophenyl)propane, 2-bromo-2-(p-nitrophenyl)-propane, 2-chloro-2-(p-cyanophenyl)propane, 2-bromo-2-(p-cyanophenyl)propane, 2-chloro-2-(p-methylcarbonylphenyl)propane, 2-bromo-2-(p-methylcarbonylphenyl)propane, 2-chloro-2-(p-phenylcarbonylphenyl)propane, 2-bromo-2-(p-phenylcarbonylphenyl)-propane, 2-chloro-2-(p-methoxycarbonylphenyl)propane, 2-bromo-2-(p-methoxycarbonylphenyl)propane, 2-chloro-1-(p-phenoxycarbonylphenyl)propane, 2-bromo-2-(p-phenoxycarbonylphenyl)propane, 2-chloro-2-(p-sulfonylphenyl)propane, 2-bromo-2-(p-sulfonylphenyl)propane, 2-chloro-2-(p-trifluoromethylphenyl)propane, 2-bromo-2-(p-trifluoromethylphenyl)propane, 2-(chloromethyl)pyridine, 2-(bromomethyl)pyridine, 2-(1-chloroethyl)pyridine, 2-(1-bromoethyl)pyridine, 2-(2-chloropropyl)pyridine, 2-(2-bromopropyl)pyridine, methyl 2-chloroethanoate, methyl 2-bromoethanoate, methyl 2-chloropropionate, methyl 2-bromoethanoate, methyl 2-chloro-2-methylpropionate, methyl 2-bromo-2-methylpropionate, ethyl 2-chloroethanoate, ethyl 2-bromoethanoate, ethyl 2-chloropropionate, ethyl 2-bromoethanoate, ethyl 2-chloro-2-ethylpropionate, ethyl 2-bromo-2-ethylpropionate, 2-chloroacetonitrile, 2-bromoacetonitrile, 2-chloropropionitrile, 2-bromopropionitrile, 2-chloro-2-methylpropionitrile, 2-bromo-2-methylpropionitrile, (1-bromoethyl)benzene, ethyl-2-bromo-iso-butylate, 1-(1-bromoethyl)-4-chlorobenzene, 1-(1-bromoethyl)-4-trifluoromethylbenzene, 1-(1-bromoethyl)-3,5-bis-trifluoromethylbenzene, 1,2,3,4,5-pentafluoro-6-(1-bromoethyl)benzene, 1-(1-bromoethyl)-4-methoxybenzene, and ethyl-2-bromo-isobutylate.

Examples of compounds represented by the formula (3) are as follows.

M(R¹)m (3)

wherein R¹ is as defined above, M is an alkali metal, alkaline earth metal or copper atom, and m is 1 when M is an alkali metal, m is 2 when M is an alkaline earth metal, or m is 1 or 2 when M is a copper atom.

Examples of groups represented by R¹ are as given above.

Examples of metals represented by M are lithium, sodium, potassium and like alkali metals, magnesium, calcium and like alkaline earth metals, and copper. Lithium is desirable.

In case that M is magnesium, the compound (3) may either be Mg(R¹)₂ or a compound represented by MgX (X is a halogen atom) which is a Grignard reagent. Chlorine and bromine are preferable.

Examples of compounds usable are methyllithium, ethyllithium, n-butyllithium, phenyllithium, p-chlorophenyllithium, p-methoxyphenyllithium and p-nitrophenyllithium.

Methyllithium, ethyllithium and n-butyllithium are preferable.

Next, a detailed description will be given of the process for preparing the compound.

Metallic tellurium is suspended in a solvent. Examples of solvents usable are dimethylformamide (DMF), tetrahydrofuran (THF) and like polar solvents, toluene, xylene and like aromatic solvents, hexane and like aliphatic hydrocarbons, dialkyl ethers and like ethers. THF is preferable. The amount of solvent to be used, which is suitably adjusted, is 1 to 100 ml, preferably 5 to 10 ml, per gram of metallic tellurium.

A compound (3) is slowly added dropwise to the suspension, followed by stirring. The reaction time differs with the reaction temperature and pressure and is usually 5 minutes to 24 hours, preferably 10 minutes to 2 hours. The reaction temperature is -20°C to 80°C, preferably 0°C to 40°C, more preferably room temperature. The reaction is conducted usually under atmospheric pressure, but may be conducted at increased pressure or in a vacuum.

Next, a compound (2) is added to the reaction mixture, followed by stirring. The reaction time differs with the reaction temperature and pressure and is usually 5 minutes to 24 hours, preferably 10 minutes to 2 hours. The reaction temperature is -20°C to 80°C, preferably 15°C to 40°C, more preferably room temperature. The reaction is conducted usually under atmospheric pressure, but may be conducted at increased pressure or in a vacuum.

The proportions of the compound (2) and compound (3) to metallic tellurium are 0.5 to 1.5 moles of the compound (2) and 0.5 to 1.5 moles of the compound (3), preferably 0.8 to 1.2 moles of the compound (2) and 0.8 to 1.2 moles of the compound (3), per mole of metallic tellurium.

After the completion of the reaction, the solvent is concentrated, and the desired compound is isolated and purified. Although the method of purification can be determined suitably depending on the compound, usually vacuum distillation or recrystallization is preferable.

The vinyl monomer to be used in the present invention is not particularly limited insofar as the monomer can be subjected to radical polymerization. Examples of vinyl monomers usable are methyl (meth)acrylate, ethyl (meth)acrylate, propyl (meth)acrylate, butyl (meth)acrylate, octyl (meth)acrylate, lauryl (meth)acrylate, (meth)acrylic acid 2-hydroxyethyl ester [2-hydroxyethyl (meth)acrylate] and like (meth)acrylic acid esters, cyclohexyl (meth)acrylate, methylcyclohexyl (meth)acrylate, isobornyl (meth)acrylate, cyclododecyl (meth)acrylate and like cycloalkyl-containing unsaturated monomers, (meth)acrylic acid, maleic acid, fumaric acid, itaconic acid, citraconic acid, crontonic acid, maleic anhydride and like carboxyl-containing unsaturated monomers, N,N-dimethylaminopropyl(meth)acrylamide, N,N-dimethylaminoethyl(meth)acrylamide, 2-(dimethylamino)ethyl (meth)acrylate, N,N-dimethylaminopropyl (meth)acrylate and like unsaturated monomers containing a tertiary amine, N-2-hydroxy-3-acryloyloxypropyl-N,N,N-trimethylammonium chloride, N-methacryloylaminoethyl-N,N,N-dimethylbenzylammonium chloride and like unsaturated monomers containing quaternary ammonium base, glycidyl (meth)acrylate and like epoxy-containing unsaturated monomers, styrene, α-methylstyrene, 4-methylstyrene, 2-methylstyrene, 3-methylstyrene, 4-methoxystyrene, p-t-butylstyrene, p-n-butylstyrene, p-tert-butoxystyrene, 2-hydroxymethylstyrene, 2-chlorostyrene, 4-chlorostyrene, 2,4-dichlorostyrene, 1-vinylnaphthalene, divinylbenzene, p-styrenesulfonic acid or an alkali metal salt thereof (sodium salt or potassium salt) and like aromatic unsaturated monomers (styrene type monomer), 2-vinylthiophene, N-methyl-2-vinylpyrrole, 1-vinyl-2-pyrrolidone, 2-vinylpyridine, 4-vinylpyridine and like unsaturated monomers containing a heterocyclic ring, N-vinylformaldehyde, N-vinylacetamide and like vinylamides, 1-hexane, 1-octene, 1-decene and like α-olefins, butadiene, isoprene, 4-methyl-1,4-hexadiene, 7-methyl-1,6-octadiene and like dienes, methyl vinyl ketone, ethyl vinyl ketone and like unsaturated monomers containing a carbonyl group, vinyl acetate, vinyl benzoate, hydroxyethyl (meth)acrylate, (meth)acrylonitrile, (meth)acrylamide, N-methyl(meth)acrylamide, N-isopropyl-(meth)acrylamide, N,N-dimethyl(meth)acrylamide and like (meth)acrylamide type monomers, vinyl chloride, and vinylidene chloride.

Preferable among these are (meth)acrylic acid ester monomers, unsaturated monomers containing a tertiary amine, aromatic unsaturated monomers (styrene type monomers), unsaturated monomers containing a carboxyl group, unsaturated monomers containing a carbonyl group, acrylamide, (meth)acrylamide and N,N-dimethylacrylamide. Particularly preferable are (meth)acrylic acid ester monomers, aromatic unsaturated monomers (styrene type monomers), unsaturated monomers containing a carboxyl group, unsaturated monomers containing a carbonyl group, (meth)acrylonitrile, (meth)acrylamide type monomers.

Examples of preferable (meth)acrylic acid ester monomers are methyl (meth)acrylate, ethyl (meth)acrylate, propyl (meth)acrylate, butyl (meth)acrylate and (meth)acrylic acid 2-hydroxyethyl ester [2-hydroxyethyl (meth)acrylate]. Especially preferable are methyl (meth)acrylate and butyl (meth)acrylate. Among these preferable are methyl methacrylate, ethyl methacrylate, propyl methacrylate, butyl methacrylate and methacrylic acid 2-hydroxyethyl ester [2-hydroxyethyl methacrylate].

Examples of preferable unsaturated monomers containing a tertiary amine are N,N-dimethylaminoethyl(meth)acrylamide and 2-(dimethylamino)ethyl (meth)acrylate.

Examples of preferable styrene type monomers are styrene, α -methylstyrene, o-methylstyrene, p-methylstyrene, p-methoxystyrene, p-t-butylstyrene, p-n-butylstyrene, p-tert-butoxystyrene, p-chlorostyrene, and p-styrenesulfonic acid or an alkali metal salt thereof (sodium salt or potassium salt). More preferable are styrene, p-methoxysytrene and p-chlorostyrene.

Example of preferable unsaturated monomer containing a carboxyl group is (meth)acrylic acid. Examples of preferable (meth)acrylamide type monomers are dimethylacrylamide and N-isopropylacrylamide. Preferable diene monomer is isoprene. Example of preferable unsaturated monomers containing a heterocyclic ring are 1-vinyl-2-pyrrolidone, 2-vinylpyridine and 4-vinylpyridine

The term "(meth)acrylic acid" refers collectively to "acrylic acid" and "methacrylic acid."

An azo type polymerization initiator used in the present invention are not particularly limited insofar as it is usable in a usual radical polymerization. Example thereof are 2,2'-azobis-isobutyronitrile (AIBN), 2,2'-azobis-2-methylbutyronitrile (AMBN), 2,2'-azobis-2,4-dimethylvaleronitrile (ADVN), 1,1'-azobis-1-cyclohexanecarbonitrile (ACHN), dimethyl-2,2'-azobisisobutyrate (MAIB), 4,4'-azobis-4-cyanovaleric acid (ACVA), 1,1'-azobis-(1-acetoxy-1-phenylethane), 2,2'-azobis (4-methoxy-2,4-dimethylvaleronitrile), 2,2'-azobis (2,4,4-trimethylpentane) and 2-cyano-2-propylazoformamide.

Specifically stated, the living radical polymer of the present invention is produced by the process to be described below.

A vinyl monomer, an organotellurium compound represented by the formula (1) and an azo type polymerization initiator are mixed together in a container having its inside air replaced by an inert gas. At this time, the organotellurium compound represented by the formula (1) and the azo type polymerization initiator may be mixed together by stirring as the first step, followed by the second step of adding the vinyl monomer to the mixture. Examples of inert gases usable at this time are nitrogen, argon and helium, among which argon and nitrogen are preferred. Nitrogen is especially preferred.

Although the vinyl monomer and the organotellurium compound represented by the formula (1) are used in amounts which are suitably adjusted depending on the molecular weight and molecular weight distribution of the living radical polymer to be obtained, usually 5 to 10,000 moles, preferably 50 to 5,000 moles, of the vinyl monomer is used per mole of the organotellurium compound represented by the formula (1).

The organotellurium compound represented by the formula (1) and the azo type polymerization initiator are used in the ratio of usually 0.1 to 100 moles, preferably 0.5 to 100 moles, more preferably 1 to 10 moles, especially preferably 1 to 5 moles, of the azo type polymerization initiator per mole of the organotellurium compound of the formula (1).

The polymerization reaction is conducted usually in the absence of solvent, while an organic solvent generally in use for radical polymerization may be used. Examples of solvents usable are benzene, toluene, N,N-dimethylformamide (DMF), dimethyl sulfoxide (DMSO), acetone, chloroform, carbon tetrachloride, tetrahydrofuran (THF), ethyl acetate and trifluoromethylbenzene. Aqueous solvents are also usable which include, for example, water, methanol, ethanol, isopropanol, n-butanol, ethyl cellosolve, butyl cellosolve, and 1-methoxy-2-propanol. The amount of the solvent to be used is adjusted suitably. For example, 0.01 to 100 ml, preferably 0.05 to 10 ml, more preferably 0.05 to 0.5 ml, of the solvent is used per gram of the vinyl monomer.

Next, the mixture is then stirred. The reaction temperature and the reaction time may be adjusted suitably in accordance with the molecular weight or molecular weight distribution of the living radical polymer to be obtained. The mixture is stirred usually at 20 to 150°C for 1 minute to 50 hours, preferably at 20 to 100°C for 0.1 to 15 hours. The mixture is stirred more preferably at 20 to 80°C for 0.1 to 5 hours. Thus, the present invention has a feature that a high yield and precise PD are performed even at such a low polymerization temperature and short period of polymerization time. The reaction is conducted usually under atmospheric pressure, but may be conducted at increased pressure or in a vacuum.

After the completion of the reaction, the solvent used and the remaining monomer are removed in a vacuum to take out the desired polymer, or the desired product is isolated by re-precipitation using a solvent wherein the product is insoluble. The reaction mixture can be treated by any method insofar as it causes no problem to the desired product.

Different kinds of vinyl monomers are usable in the process of the invention for preparing a living radical polymer. For example when at least two kinds of vinyl monomers are reacted at the same time, a random copolymer can be obtained. The random copolymer obtained is a polymer which comprises the reacted monomers in the original ratio (mole ratio) regardless of the kinds of the monomers. When a random copolymer is obtained by reacting a vinyl monomer A and a vinyl monomer B at the same time, the copolymer has substantially the same material ratio (mole ratio). Further when two kinds of vinyl monomers are reacted in succession, a block copolymer can be obtained. The block copolymer is provided by the same order of reacted monomers regardless of the kinds of the monomers. If a vinyl monomer A and a vinyl monomer B are reacted in succession to obtain a block copolymer, the polymer obtained is in the order of A-B or B-A in conformity with the order of monomers reacted.

The living radical polymerization initiator of the present invention is adapted for excellent control of molecular weights and molecular weight distributions under very mild conditions. In particular, the present polymerization reaction proceeds in a shortened reaction time than the conventional living radical polymerization reaction.

The living radical polymer to be obtained by the invention is adjustable in molecular weight according to the reaction time and the amount of the organotellurium compound, and can be 500 to 1,000,000 in number average molecular weight. The invention is especially suitable for producing living radical polymers having a number average molecular weight of 1,000 to 50,000.

The living radical polymer to be obtained by the invention is controlled to 1.02 to 1.50 in molecular weight distribution (PD = Mw/Mn). The molecular weight distribution is controllable to a narrower range of 1.02 to 1.30, a further narrower range of 1.02 to 1.20, a still narrower range of 1.02 to 1.10.

It has been found that the living radical polymer of the present invention has a terminal group which is an alkyl, substituted aryl, aromatic heterocyclic group, acyl, oxycarbonyl or cyano derived from the organotellurium compound and a growth terminal which is highly reactive tellurium. Accordingly, the organotellurium compound used for radical polymerization makes it easier to convert the terminal group to other functional group than in the case of the living radical polymer obtained by conventional living radical polymerization. The living radical polymer obtained according to the invention is therefore usable as a macro living radical polymerization initiator (macroinitiator).

A-B diblock copolymers such as methyl methacrylate-styrene and B-A diblock copolymers such as styrene-methyl methacrylate can be obtained using a macro living radical polymerization initiator of the invention. A-B-A triblock copolymers such as methyl methacrylate-styrene-methyl methacrylate and A-B-C triblock copolymers such as methyl methacrylate-styrene-butyl acrylate are also available. This is attributable to the fact that the vinyl monomers of various different types are controllable by the organotellurium compound and the azo type polymerization initiator of the invention, and also to the fact that highly reactive tellurium is present at the growth terminal of the living radical polymer obtained with use of the living radical polymerization initiator.

Stated more specifically, block copolymers are prepared by the processes to be described below.

For preparing A-B diblock copolymers such as methyl methacrylate-styrene copolymer, methyl methacrylate, a living radical polymerization initiator represented by the formula (1) and an azo type polymerization initiator are mixed together first as in the process described above for preparing a living radical polymer to obtain poly(methyl methacrylate), and subsequently mixing styrene with the polymer to obtain methyl methacrylate-styrene copolymer.

A-B-A triblock copolymers and A-B-C triblock copolymers can be produced, for example, by preparing an A-B diblock copolymer by the above process and thereafter mixing a vinyl monomer (A) or vinyl monomer (C) with the copolymer to obtain the A-B-A or A-B-C triblock copolymer.

In producing the diblock copolymer according to the invention, the organotellurium compound of the formula (1) and the azo type polymerization initiator can be used when a homopolymer is prepared from the first monomer and/or when the diblock copolymer is subsequently prepared.

Further in producing the triblock copolymer according to the invention the organotellurium compound of the formula (1) and the azo type polymerization initiator can be used at least once when a homopolymer is prepared from the first monomer, or when a diblock copolymer is subsequently prepared, or when the triblock copolymer is subsequently prepared.

The preparation of each block may be followed directly by the subsequent reaction for the next block, or the subsequent reaction for the next block may be initiated after the purification of the product resulting from the completion of the first reaction. The block copolymer can be isolated by a usual method.

### BEST MODE OF CARRYING OUT THE INVENTION

The present invention will be described below in detail with reference to Examples, but is not limited thereto in any way. In Examples and Comparative Examples, properties were determined by the following methods.

### (1) Identification of organotellurium compounds and living radical polymers

The organotellurium compound was identified based on the results of ¹H-NMR, ¹³C-NMR, IR and MS analyses. The molecular weight and molecular weight distribution of the living radical polymer were determined using GPC (gel permeation chromatography). The measuring instruments used are as follows.
¹H-NMR: Varian Gemini 2000 (300MHz for ¹H), JEOL JNM-A400 (400MHz for ¹H)
¹³C-NMR : Varian Gemini 2000, JEOL JNM-A400
IR: Shimadzu FTIR-8200 (cm⁻¹)
MS (HRMS): JEOL JMS-300
Molecular weight and molecular weight distribution : liquid chromatography Shimadzu LC-10 [column: Shodex K-804L + K-805L, polystyrene standard : TOSOH TSK Standard, poly(methyl methacrylate) standard : Shodex Standard M-75]

The molecular weight and molecular weight distribution of the desired polymer in Examples 20 and 21 were determined using the following liquid chromatography.

Tosoh Corporation, HLC-8220GPC [column: TSK-GEL α-6000 + TSK-GEL α-4000 + TSK-GEL α-2500 polyethylene oxide standard : TSK Standard POLY(ETHYLENE OXIDE)]

### Preparation Example 1

### Preparation of (1-methyltellanyl-ethyl)benzene

A 6.38 g quantity (50 mmoles) of metallic tellurium [product of Aldrich, brand name: Tellurium (-40 mesh)] was suspended in 50 ml of THF, and 52.9 ml (1.04 M diethyl ether solution, 55 mmoles) of methyllithium (product of Kanto Chemical Co., Ltd., diethyl ether solution) was slowly added dropwise to the suspension at room temperature (for 10 minutes). The reaction mixture was stirred until the metallic tellurium disappeared completely (for 20 minutes). To the reaction mixture was added 11.0 g (60 mmoles) of (1-bromoethyl)benzene at room temperature, followed by stirring for 2 hours. After the completion of reaction, the solvent was concentrated in a vacuum, followed by vacuum distillation to give 8.66 g of yellow oil (70% in yield).

IR, HRMS, ¹H-NMR and ¹³C-NMR analyses indicated that the product was (1-methyltellanyl-ethyl)benzene.
IR(neat, cm⁻¹) 1599, 1493, 1451, 1375, 1219, 1140, 830, 760, 696, 577
HRMS (EI) m/z: Calcd for C₉H₁₂Te(M)⁺, 250.0001; Found 250.0001 ¹H-NMR (300 MHz, CDCl₃) 1.78 (s, 3H, TeCH₃), 1.90 (d, J=7.2Hz, 3H), 4.57 (q, J=7.2Hz, 1H, CHTe), 7.08-7.32 (m, 5H)
¹³C-NMR (75 MHz, CDCl₃) -18.94, 18.30, 23.89, 126.17, 126.80, 128.30, 145.79

### Preparation Example 2

### Preparation of ethyl-2-methyl-2-methyltellanyl-propionate

The same procedure as in Preparation Example 1 was performed with the exception of using 10.7 g (55 mmoles) of ethyl-2-bromo-isobutyrate in place of (1-bromoethyl-)benzene to obtain 6.53 g (51% in yield) of yellow oil.
IR, HRMS, ¹H-NMR and ¹³C-NMR analyses indicated that the product was ethyl-2-methyl-2-methyltellanyl-propionate. IR(neat, cm⁻¹) 1700, 1466, 1385, 1269, 1146, 1111, 1028
HRMS (EI) m/z: Calcd for C₇H₁₄O₂Te(M)⁺, 260.0056; Found 260.0053
¹H-NMR (300 MHz, CDCl₃) 1.27 (t, J=6.9Hz, 3H), 1.74 (s, 6H), 2.15 (s, 3H, TeCH₃), 4.16 (q, J=7.2Hz, 2H)
¹³C-NMR (75 MHz, CDCl₃) -17.38, 13.89, 23.42, 27.93, 60.80, 176.75

### Preparation Example 3

### Preparation of 2-methyl-2-methyltellanyl-propionitrile

The same procedure as in Preparation Example 1 was performed with the exception of using 10.4 g (70 mmoles) of 2-bromo-2-methyl-propionitrile in place of (1-bromoethyl)benzene to obtain 4.10 g (39% in yield) of red oil.

IR, HRMS, ¹H-NMR and ¹³C-NMR analyses indicated that the product was 2-methyl-2-methyltellanyl-propionitrile. IR(neat, cm⁻¹) 2217, 1713, 1458, 1370, 1225, 1117, 835 HRMS(EI)m/z: Calcd for C₅H₉NTe(M)⁺, 212.9797; Found 212.9799 ¹H-NMR (300MHz, CDCl₃) 1.91(s, 6H), 2.38(s, 3H, TeCH₃) ¹³C-NMR (75MHz, CDCl₃) -15.5, 2.2, 30.3, 125.1

### Preparation Example 4

### Preparation of ethyl-2-methyl-2-n-butyltellanyl-propionate

The same procedure as in Preparation Example 1 was performed with the exception of using 34.4 ml (55 mmoles) of n-butyllithium [product of Aldrich, 1.6 M hexane solution] in place of methyllithium and 10.7 g (55 mmoles) of ethyl-2-bromo-isobutyrate in place of (1-bromoethyl)benzene to obtain 8.98 g (59.5% in yield) of yellow oil.
¹H-NMR analysis indicated that the product was ethyl-2-methyl-2-n-butyltellanyl-propionate.
¹H-NMR (300MHz, CDCl₃) 0.93(t, J=7.5Hz, 3H), 1.25(t, J=7.2Hz, 3H), 1.37(m, 2H), 1.74(s, 6H), 1.76(m, 2H) 2.90(t, J=7.5Hz, 2H, CH₂Te), 4.14(q, J=7.2Hz, 2H)

### Reference Example 1

2-Bromo-2-methyl-propionitrile which was used in Preparation Example 3 was prepared as follows.

Bromine was slowly added dropwise to a solution of isobutyronitrile (200 mmoles) and phosphorus tribromide (PBr₃, 200 mmoles) in ether (Et₂O, 100 ml) in a reaction vessel while cooling with an ice-bath. After completion of dropwise addition, the mixture was reacted at room temperature for 14 hours. The resulting solution was poured into ice-water for work up, extracted with ether (three times), dried with magnesium sulfate, filtered to remove magnesium sulfate. The filtrate was concentrated by removing solvent by an evaporator. The resulting concentrate was purified by distillation, giving 17.08 g of colorless transparent liquid (b.p. 57°C / 43 mmHg) in 58 % yield.

### Example 1

Along with 2-methyl-2-methyltellanyl-propionitrile prepared in Preparation Example 3 (0.10 mmole), 2,2'-azo-bisisobutyronitrile (Otsuka Chemical Co., Ltd., AIBN)(0.10 mmole) and styrene (St, 10 mmoles) were placed into a glove box with the inside air replaced by nitrogen, followed by stirring. After the completion of the reaction, the reaction mixture was dissolved in 5 ml of chloroform, and the solution was then poured into 250 ml of hexane which was being stirred. The resulting polymer precipitate was collected by suction filtration and dried to obtain a polymer.

Table 1 shows the result of GPC analysis (with reference to the molecular weight of an authentic sample of polystyrene].

### Comparative Example 1

A polystyrene was prepared in the same manner as in Example 1 except that no azo type polymerization initiator is used and the reaction was conducted at 105°C for 18 hours. Table 1 shows the result.

**Table 1**

| | monomer | reaction condition | yield (%) | Mn | PD |
|---|---|---|---|---|---|
| Ex.1 | St | 60 °C, 11 h | 94 | 11300 | 1.13 |
| Com. Ex. 1 | St | 105 °C, 18 h | 96 | 9200 | 1.17 |

### Example 2 and Comparative Example 2

Polymerizations were conducted in the same manner as in Example 1 and Comparative Example 1, except that n-butyl acrylate (nBA) was used in place of styrene, respectively. GPC analysis was carried out with reference to the molecular weight of an authentic sample of poly(methyl methacrylate). Table 2 shows the result.

**Table 2**

| | monomer | reaction condition | yield (%) | Mn | PD |
|---|---|---|---|---|---|
| Ex.2 | nBA | 60 °C, 4 h | 99 | 15900 | 1.12 |
| Com. Ex.2 | nBA | 100 °C, 24 h | 89 | 10300 | 1.13 |

As apparent from Examples and Comparative Examples, when an azo type polymerization initiator is used as in the present invention, living radical polymers having narrow molecular weight distributions (PD value is more close to 1) are obtained under mild conditions and in a short period of reaction time.

### Example 3

Polymerization was conducted in the same manner as in Example 1, except that (1-methyltellanyl-ethyl)benzene prepared in Preparation Example 1 was used in place of 2-methyl-2-methyltellanyl-propionitrile. GPC analysis was carried out with reference to the molecular weight of an authentic sample of polystyrene. Result is shown below.
Monomer: Styrene
Reaction condition: 60°C, 11 hours
Yield: 91 %
Mn: 9100
PD: 1.17

### Example 4

Polymerization was conducted in the same manner as in Example 1, except that ethyl-2-methyl-2-methyltellanyl-propionate prepared in Preparation Example 2 was used in place of 2-methyl-2-methyltellanyl-propionitrile. GPC analysis was carried out with reference to the molecular weight of an authentic sample of polystyrene. Result is shown below.
Monomer: Styrene
Reaction condition: 60 °C, 11 hours
Yield: 92 %
Mn: 11500
PD: 1.17

### Example 5

A poly(n-butyl acrylate) having Mn=16200 and PD=1.14 was prepared in 99 % yield in the same manner as in Example 2 except that the reaction was conducted at 70 °C for 0.25 hour. Table 1 shows the result.

### Example 6

Along with ethyl-2-methyl-2-methyltellanyl-propionate prepared in Preparation Example 2 (0.20 mmole), 2-cyano-2-propylazoformamide (Wako Pure Chemical Industries, Ltd., V-30)(0.10 mmole) and isoprene (Wako Pure Chemical Industries, Ltd., 20 mmoles) were placed into a glove box with the inside air replaced by nitrogen, followed by stirring at 100°C for 24 hours. After the completion of the reaction, the remaining monomer was removed under the decompression to obtain a polyisoprene in 79 % yield. GPC analysis (with reference to the molecular weight of an authentic sample of polystyrene) revealed Mn 6300 and PD=1.46.

### Example 7

Along with ethyl-2-methyl-2-methyltellanyl-propionate prepared in Preparation Example 2 (0.10 mmole), 2,2'-azo-bisisobutyronitrile (Otsuka Chemical Co., Ltd., AIBN)(0.10 mmole) and 1-vinyl-2-pyrrolidone (Wako Pure Chemical Industries, Ltd., 10 mmoles) were placed into a glove box with the inside air replaced by nitrogen, followed by stirring at 60°C for 1 hour. After the completion of the reaction, the reaction mixture was dissolved in 5 ml of chloroform, and the solution was then poured into 250 ml of hexane which was being stirred. The resulting polymer precipitate was collected by suction filtration and dried to obtain a poly(1-vinyl-2-pyrrolidone) in 99 % yield. GPC analysis (with reference to the molecular weight of an authentic sample of polystyrene) revealed Mn 41400 and PD=1.14.

### Example 8

Along with ethyl-2-methyl-2-methyltellanyl-propionate prepared in Preparation Example 2 (0.10 mmole), 4,4'-azo-bis-4-cyanovaleric acid (Otsuka Chemical Co., Ltd., ACVA)(0.05 mmole), 1-vinyl-2-pyrrolidone (Wako Pure Chemical Industries, Ltd., 10 mmoles) and deaerated pure water (1 ml) were placed into a glove box with the inside air replaced by nitrogen, followed by stirring at 60°C for 1 hour. After the completion of the reaction, water was removed under the decompression and the residue was dissolved in 5 ml of chloroform, and the solution was then poured into 250 ml of hexane which was being stirred. The resulting polymer precipitate was collected by suction filtration and dried to obtain a poly(1-vinyl-2-pyrrolidone) in 99 % yield. GPC analysis (with reference to the molecular weight of an authentic sample of polystyrene) revealed Mn 33200 and PD=1.03.

### Example 9

Along with ethyl-2-methyl-2-methyltellanyl-propionate prepared in Preparation Example 2 (0.10 mmole), 2,2'-azo-bisisobutyronitrile (Otsuka Chemical Co., Ltd., AIBN)(0.10 mmole) and p-tert-butoxystyrene (Wako Pure Chemical Industries, Ltd., 10 mmoles) were placed into a glove box with the inside air replaced by nitrogen, followed by stirring at 60 °C for 13 hours. After the completion of the reaction, the reaction mixture was dissolved in 5 ml of chloroform, and the solution was then poured into 250 ml of methanol which was being stirred. The resulting polymer precipitate was collected by suction filtration and dried to obtain a poly(p-tert-butoxystyrene) in 92 % yield. GPC analysis (with reference to the molecular weight of an authentic sample of polystyrene) revealed Mn 14300 and PD=1.15.

### Example 10

Along with ethyl-2-methyl-2-methyltellanyl-propionate prepared in Preparation Example 2 (0.10 mmole), 2,2'-azo-bisisobutyronitrile (Otsuka Chemical Co., Ltd., AIBN)(0.05 mmole) and 2-vinylpyridine (Wako Pure Chemical Industries, Ltd., 10 mmoles) were placed into a glove box with the inside air replaced by nitrogen, followed by stirring at 60 °C for 3 hours. After the completion of the reaction, the reaction mixture was dissolved in 5 ml of chloroform, and the solution was then poured into 250 ml of hexane which was being stirred. The resulting polymer precipitate was collected by suction filtration and dried to obtain a poly(2-vinylpyridine) in 99 % yield. GPC analysis (with reference to the molecular weight of an authentic sample of polystyrene) revealed Mn 32200 and PD=1.18.

### Example 11

Along with ethyl-2-methyl-2-methyltellanyl-propionate prepared in Preparation Example 2 (0.10 mmole), 2,2'-azo-bisisobutyronitrile (Otsuka Chemical Co., Ltd., AIBN)(0.05 mmole) and 4-vinylpyridine (Wako Pure Chemical Industries, Ltd., 10 mmoles) were placed into a glove box with the inside air replaced by nitrogen, followed by stirring at 60°C for 2 hours. After the completion of the reaction, the reaction mixture was dissolved in 5 ml of chloroform, and the solution was then poured into 250 ml of hexane which was being stirred. The resulting polymer precipitate was collected by suction filtration and dried to obtain a poly(4-vinylpyridine) in 94 % yield. GPC analysis (with reference to the molecular weight of an authentic sample of polystyrene) revealed Mn 35800 and PD=1.10.

### Example 12

Along with ethyl-2-methyl-2-methyltellanyl-propionate prepared in Preparation Example 2 (0.10 mmole), 2,2'-azobis(4-methoxy-2,4-dimethylvaleronitrile) (Wako Pure Chemical Industries, Ltd., V-70)(0.10 mmole) and styrene (Wako Pure Chemical Industries, Ltd., 10 mmoles) were placed into a glove box with the inside air replaced by nitrogen, followed by stirring at 40°C for 23 hours. After the completion of the reaction, the reaction mixture was dissolved in 5 ml of chloroform, and the solution was then poured into 250 ml of methanol which was being stirred. The resulting polymer precipitate was collected by suction filtration and dried to obtain a polystyrene in 82 % yield. GPC analysis (with reference to the molecular weight of an authentic sample of polystyrene) revealed Mn 7400 and PD=1.21.

### Example 13

Along with ethyl-2-methyl-2-methyltellanyl-propionate prepared in Preparation Example 2 (0.10 mmole), 2,2'-azobis(4-methoxy-2,4-dimethylvaleronitrile) (Wako Pure Chemical Industries, Ltd., V-70)(0.10 mmole) and n-butyl acrylate (Wako Pure Chemical Industries, Ltd., 10 mmoles) were placed into a glove box with the inside air replaced by nitrogen, followed by stirring at 40°C for 2 hours. After the completion of the reaction, the reaction mixture was dissolved in 5 ml of chloroform, and the solution was then poured into 250 ml of hexane which was being stirred. The resulting polymer precipitate was collected by suction filtration and dried to obtain a poly(n-butyl acrylate) in 96 % yield. GPC analysis [with reference to the molecular weight of an authentic sample of poly(methyl methacrylate)] revealed Mn 12800 and PD=1.17.

### Example 14

Along with ethyl-2-methyl-2-methyltellanyl-propionate prepared in Preparation Example 2 (0.10 mmole), 2,2'-azobis(4-methoxy-2,4-dimethylvaleronitrile) (Wako Pure Chemical Industries, Ltd., V-70)(0.10 mmole) and methyl methacrylate (Wako Pure Chemical Industries, Ltd., 10 mmoles) were placed into a glove box with the inside air replaced by nitrogen, followed by stirring at 40°C for 3 hours. After the completion of the reaction, the reaction mixture was dissolved in 5 ml of chloroform, and the solution was then poured into 250 ml of hexane which was being stirred. The resulting polymer precipitate was collected by suction filtration and dried to obtain a poly(methyl acrylate) in 82 % yield. GPC analysis [with reference to the molecular weight of an authentic sample of poly(methyl methacrylate)] revealed Mn 10900 and PD=1.17.

### Example 15

Along with ethyl-2-methyl-2-methyltellanyl-propionate prepared in Preparation Example 2 (0.10 mmole), 2,2'-azo-bisisobutyronitrile (Otsuka Chemical Co., Ltd., ACVA)(0.10 mmole), acrylonitrile (Wako Pure Chemical Industries, Ltd., 10 mmoles) and N-dimethylformamide (DMF, 1.35 ml) were placed into a glove box with the inside air replaced by nitrogen, followed by stirring at 60°C for 3 hours. After the completion of the reaction, solvent and remaining monomer were removed under the decompression using vacuum pump to obtain a polyacrylonitrile in 99 % yield. GPC analysis (with reference to the molecular weight of an authentic sample of polystyrene) revealed Mn 37800 and PD=1.16.

### Example 16

Along with ethyl-2-methyl-2-methyltellanyl-propionate prepared in Preparation Example 2 (0.10 mmole), 2,2'-azo-bisisobutyronitrile (Otsuka Chemical Co., Ltd., AIBN)(0.10 mmole) and N,N-dimethylacrylamide (Wako Pure Chemical Industries, Ltd., 10 mmoles) were placed into a glove box with the inside air replaced by nitrogen, followed by stirring at 60 °C for 6 hours. After the completion of the reaction, the reaction mixture was dissolved in 5 ml of chloroform, and the solution was then poured into 250 ml of hexane which was being stirred. The resulting polymer precipitate was collected by suction filtration and dried to obtain a poly(N,N-dimethylacrylamide) in 99 % yield. GPC analysis [with reference to the molecular weight of an authentic sample of poly(methyl methacrylate)] revealed Mn 17400 and PD=1.18.

### Example 17

Along with ethyl-2-methyl-2-methyltellanyl-propionate prepared in Preparation Example 2 (0.10 mmole), 2,2'-azo-bisisobutyronitrile (Otsuka Chemical Co., Ltd., AIBN)(0.10 mmole), N-isopropylacrylamide (Wako Pure Chemical Industries, Ltd., 10 mmoles) and DMF (1 ml) were placed into a glove box with the inside air replaced by nitrogen, followed by stirring at 60 °C for 6 hours. After the completion of the reaction, the reaction mixture was dissolved in 5 ml of chloroform, and the solution was then poured into 250 ml of hexane which was being stirred. The resulting polymer precipitate was collected by suction filtration and dried to obtain a poly(N-isopropylacrylamide) in 99 % yield. GPC analysis [with reference to the molecular weight of an authentic sample of poly(methyl methacrylate)] revealed Mn 13800 and PD=1.16.

### Example 18

Along with ethyl-2-methyl-2-methyltellanyl-propionate prepared in Preparation Example 2 (0.10 mmole), 2,2'-azo-bisisobutyronitrile (Otsuka Chemical Co., Ltd., ACVA)(0.05 mmole), acrylic acid (Wako Pure Chemical Industries, Ltd., 5 mmoles) and tetrahydrofuran (THF, 0.5 ml) were placed into a glove box with the inside air replaced by nitrogen, followed by stirring at 60°C for 1 hour. After the completion of the reaction, solvent and remaining monomer were removed under the decompression using vacuum pump to obtain a polyacrylic acid in 99 % yield.

Molecular weight analysis was conducted after converting carboxylic acid to corresponding methyl ester. Namely, the resulting polymer was dissolved in THF (7 ml) and trimethylsilyldiazomethane (Aldrich Corp., 5.5 mmoles) was added thereto and the mixture was stirred at room temperature for 1 hour. The solvent was removed under the decompression to obtain poly(methyl acrylate).

GPC analysis [with reference to the molecular weight of an authentic sample of poly(methyl methacrylate)] revealed Mn 7500 and PD=1.35.

### Example 19

Along with 2-methyl-2-methyltellanyl-propionitrile prepared in Preparation Example 3 (0.10 mmole), 2,2'-azo-bisisobutyronitrile (Otsuka Chemical Co., Ltd., AIBN)(0.10 mmole) and 1-vinyl-2-pyrrolidone (Wako Pure Chemical Industries, Ltd., 10 mmoles) were placed into a glove box with the inside air replaced by nitrogen, followed by stirring at 80°C for 0.5 hour. After the completion of the reaction, the reaction mixture was dissolved in 5 ml of chloroform, and the solution was then poured into 250 ml of hexane which was being stirred. The resulting polymer precipitate was collected by suction filtration and dried to obtain a poly(1-vinyl-2-pyrrolidone) in 99 % yield.

GPC analysis (with reference to the molecular weight of an authentic sample of polystyrene) revealed Mn 18000 and PD=1.19.

### Example 20

Along with ethyl-2-methyl-2-n-butyltellanyl-propionate prepared in Preparation Example 4 (0.047 mmole), AIBN (0.047 mmole), acrylamide (Wako Pure Chemical Industries, Ltd., 43.1 mmoles), deaerated pure water (15 ml) and methanol (10 ml) were placed into a glove box with the inside air replaced by nitrogen, followed by stirring at 60°C for 20 hours. After the completion of the reaction, the reaction mixture was poured into 250 ml of methanol. The resulting polymer precipitate was collected by suction filtration and dried to obtain a polyacrylamide in 72 % yield.

GPC analysis (with reference to the molecular weight of an authentic sample of polyethylene oxide) revealed Mn 65000 and PD=1.21.

### Example 21

Along with ethyl-2-methyl-2-n-butyltellanyl-propionate prepared in Preparation Example 4 (0.047 mmole), AIBN (0.047 mmole), acrylamide (Wako Pure Chemical Industries, Ltd., 86 mmoles), deaerated pure water (30 ml) and methanol (21 ml) were placed into a glove box with the inside air replaced by nitrogen, followed by stirring at 60 °C for 20 hours. After the completion of the reaction, the reaction mixture was poured into 500 ml of methanol. The resulting polymer precipitate was collected by suction filtration and dried to obtain a polyacrylamide in 75 % yield.

GPC analysis (with reference to the molecular weight of an authentic sample of polyethylene oxide) revealed Mn 102000 and PD=1.27.

### INDUSTRIAL APPLICABILITY

The present living radical polymer has precisely controlled molecular weights and molecular weight distributions (PD=Mw/Mn), and is useful for preparing a novel high polymer materials.

## Claims

1. A process for producing a living radical polymer **characterized in that** a vinyl monomer is polymerized with use of an organotellurium compound represented by the formula (1) and an azo type polymerization initiator 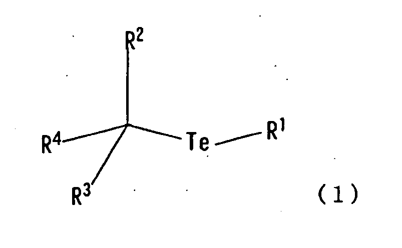 wherein R¹ is C₁-C₈ alkyl, substituted aryl or an aromatic heterocyclic group, R² and R³ are each a hydrogen atom or C₁-C₈ alkyl, and R⁴ is aryl, substituted aryl, an aromatic heterocyclic group, acyl, oxycarbonyl or cyano.

2. A process for producing a living radical polymer according to claim 1 wherein the polymerization temperature is 40 to 100°C and the polymerization time is 1 to 15 hours.

3. A process for producing a living radical polymer according to claim 2 wherein the polymerization temperature is 40 to 80°C and the polymerization time is 1 to 5 hours.

4. A process for producing a living radical polymer according to claim 1 wherein R⁴ in the organotellurium compound of the formula (1) is aryl, oxycarbonyl or cyano.

5. A process for producing a living radical polymer according to any one of claims 1 to 4 wherein the vinyl monomer is at least one selected from the group consisting of (meth)acrylic acid ester monomer, unsaturated monomer containing a carboxyl group, unsaturated monomer containing a tertiary amine, aromatic unsaturated monomer, unsaturated monomer containing a heterocyclic ring, dienes selected from the group consisting of butadiene, isoprene, 4-methyl-1,4-hexadiene and 7-methyl-1,6-octadiene, (meth)acrylonitrile, (meth)acrylamide, N-methyl(meth)acrylamide, N-isopropyl-(meth)acrylamide and N,N-dimethyl(meth)acrylamide.

## Patentansprüche

1. Verfahren zum Erzeugen eines lebenden Radikalpolymers, **dadurch gekennzeichnet, dass** ein Vinylpolymer unter Verwendung einer Organotellurverbindung nach Formel (1) und einem Polymerisationsstarter vom Azotyp polymerisiert wird, 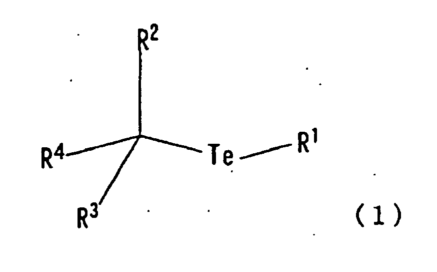 wobei R¹ C₁-C₈-Alkyl, substituiertes Aryl oder eine aromatische heterozyklische Gruppe ist, R² und R³ jeweils ein Wasserstoffatom oder C₁-C₈-Alkyl sind, und R⁴ Aryl, substituiertes Aryl, eine aromatische heterozyklische Gruppe, Acyl Oxycarbonyl oder Cyano ist.

2. Verfahren zum Erzeugen eines lebenden Radikalpolymers nach Anspruch 1, wobei die Polymerisationstemperatur 40 bis 100 °C ist und die Polymerisationsdauer 1 bis 15 Stunden ist.

3. Verfahren zum Erzeugen eines lebenden Radikalpolymers nach Anspruch 2, wobei die Polymerisationstemperatur 40 bis 80°C ist und die Polymerisationsdauer 1 bis 5 Stunden ist.

4. Verfahren zum Erzeugen eines lebenden Radikalpolymers nach Anspruch 1, wobei R⁴ in der Organotellurverbindung der Formel (1) Aryl, Oxycarbonyl oder Cyano ist.

5. Verfahren zum Erzeugen eines lebenden Radikalpolymers nach einem der Ansprüche 1 bis 4, wobei das Vinylpolymer wenigstens eines ist, das ausgewählt ist aus der Gruppe bestehend aus (Meth)acrylsäureestermonomer, ungesättigtem Monomer enthaltend eine Carbonylgruppe, ungesättigtem Monomer enthaltend ein tertiäres Amin, aromatischem ungesättigtem Monomer, ungesättigtem Monomer enthaltend einen heterozyklischen Ring, Dienen ausgewählt aus der Gruppe bestehend aus Butadien, Isopren, 4-Methyl-1,4-hexadien und 7-Methyl-1,6-octadien, (Meth)acrylnitril, (Meth)acrylamid, N-Methyl(meth)acrylamid, N-Isopropyl(meth)acrylamid und N,N-Dimethyl(meth)acrylamid.

## Revendications

1. Procédé de production d'un polymère radicalaire vivant **caractérisé en ce qu'**un monomère de vinyle est polymérisé grâce à l'utilisation d'un composé d'organotellure représenté par la formule (1) et d'un initiateur de polymérisation de type azo 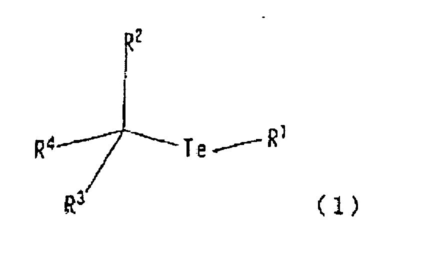 dans laquelle R¹ est un groupe alkyle en C₁-C₈, un groupe aryle substitué ou un groupe hétérocyclique aromatique, R² et R³ sont chacun un atome d'hydrogène ou un groupe alkyle en C₁-C₈, et R⁴ est un groupe aryle, un groupe aryle substitué, un groupe hétérocyclique aromatique, un groupe acyle, un groupe oxycarbonyle ou un groupe cyano.

2. Procédé de production d'un polymère radicalaire vivant selon la revendication 1, dans lequel la température de polymérisation est de 40 à 100 °C et le temps de polymérisation est de 1 à 15 heures.

3. Procédé de production d'un polymère radicalaire vivant selon la revendication 2, dans lequel la température de polymérisation est de 40 à 80 °C et le temps de polymérisation est de 1 à 5 heures.

4. Procédé de production d'un polymère radicalaire vivant selon la revendication 1, dans lequel R⁴ dans le composé d'organotellure de formule (1) est un groupe aryle, un groupe oxycarbonyle ou un groupe cyano.

5. Procédé de production d'un polymère radicalaire vivant selon l'une quelconque des revendications 1 à 4, dans lequel le monomère de vinyle est au moins un monomère choisi dans le groupe constitué par un monomère d'ester d'acide (méth)acrylique, un monomère insaturé contenant un groupe carboxyle, un monomère insaturé contenant une amine tertiaire, un monomère insaturé aromatique, un monomère insaturé contenant un cycle hétérocyclique, des diènes choisis dans le groupe constitué par le butadiène, l'isoprène, le 4-méthyl-1,4-hexadiène et le 7-méthyl-1,6-octadiène, un (méth)acrylonitrile, un (méth)acrylamide, un (méth)acrylamide de N-méthyle, un (méth)acrylamide de N-isopropyle et un (méth)acrylamide de N,N-diméthyle.
